(19) Europäisches Patentamt
European Patent Office
Office européen des brevets

(11) Publication number: **0 074 749**
**A2**

# (12) EUROPEAN PATENT APPLICATION

(21) Application number: 82304560.4

(22) Date of filing: 31.08.82

(51) Int. Cl.³: C 07 D 231/14
C 07 D 405/04, A 01 N 43/56

(30) Priority: 31.08.81 US 297700

(43) Date of publication of application:
23.03.83 Bulletin 83/12

(84) Designated Contracting States:
AT BE CH DE FR IT LI LU NL SE

(71) Applicant: UNIROYAL, INC.
1230 Avenue of the Americas Rockefeller Center
New York, New York 10020(US)

(71) Applicant: UNIROYAL LIMITED
1500 Don Mills Road
Don Mills Ontario M3B 3L4(CA)

(72) Inventor: Harris, John W.
3339 Lansdown Drive
Burlington Ontario, L7M 1S5(CA)

(72) Inventor: Harrison, William A.
34 Arthur Street North
Guelph Ontario, N1E 4T8(CA)

(72) Inventor: Bell, Allyn Roy
686 Wiese Road
Cheshire Connecticut 06410(US)

(74) Representative: Harrison, Michael Robert et al,
URQUHART-DYKES & LORD 11th Floor Tower House
Merrion Way
Leeds LS2 8PB(GB)

## (54) Substituted pyrazole herbicides.

(57) Pyrazoles useful as herbicides have the formula

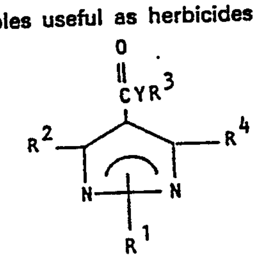

wherein $R^1$ is $C_1$-$C_4$ haloalkyl or $ROR^5$, wherein R is $C_1$-$C_2$ alkylene or $C_2$ alkylidene and $R^5$ is hydrogen, $C_1$-$C_4$ alkyl, $C_2$-$C_5$ alkanoyl, aroyl, $C_8$-$C_{10}$ aralkanoyl, $C_7$-$C_9$ aralkyl, phenyl, or phenyl substituted with $C_1$-$C_4$ alkyl, $C_1$-$C_4$ alkoxy, trihalomethyl, hydroxy, cyano or nitro; or benzoyl substituted with halogen, $C_1$-$C_4$ alkyl, $C_1$-$C_4$ alkoxy, trihalomethyl, hydroxy, cyano or nitro; $R^2$ is $C_1$-$C_4$ alkyl, $C_5$-$C_6$ cycloalkyl, $C_7$-$C_9$ aralkyl, $C_7$-$C_9$ alkaryl, phenyl, naphthyl or a 5- or 6- membered heterocyclic group containing oxygen, sulfur or nitrogen, or $R_2$ is phenyl substituted with halogen, trihalomethyl, $C_1$-$C_4$ alkoxy, hydroxy or cyano; Y is oxygen or sulfur, $R^3$ is $C_1$-$C_4$ alkyl, $C_2$-$C_4$ alkenyl, $C_3$-$C_4$ alkynyl, $C_2$-$C_6$ alkoxyalkyl or $C_2$-$C_6$ alkylthioalkyl; $R^4$ is hydrogen, $C_1$-$C_4$ alkyl or trihalomethyl; provided that $R^2$ is not 3,4-dialkylphenyl or 3,4 dihalophenyl.

Compositions including the above pyrazoles used as preemergence and postemergence treatment of weeds are also described.

EP 0 074 749 A2

## BACKGROUND OF THE INVENTION

It is known that certain pyrazole carboxylates are effective as herbicides. For example U. S. Patent No. 4,116,673 teaches phenyl substituted pyrazole-4-carboxylates, wherein the -1-position is hydrogen or alkyl substituted, as herbicides. German Patent No. 29 20 933 teaches the use of 3-aryl-5-methylpyrazole-4-carboxylates as herbicides. The substituent in the -1-position in these compounds may be hydrogen, certain alkanoyl, alkoxycarboyl or chlorophenoxycarbonyl. U.S. Patent No. 4,260,775 teaches pyrazole derivatives having an amino group in the -3- or -5- position as herbicides. Bastide et al teach the preparation of certain 3-aryl-5-methyl and 5-aryl-3-methyl pyrazole-4-carboxylates where the substituent on the -1-position is hydrogen; see "Cycloaddition of Diazoalkanes Onto Disubstituted Alkenes and Alkynes", Tetrahedron, 30, 3355-3363 Pergamon Press, 1974, Great Britain. Herbicidal activity is not taught for the composition.

## SUMMARY OF THE INVENTION

It has been surprisingly found that pyrazoles of the general formula:

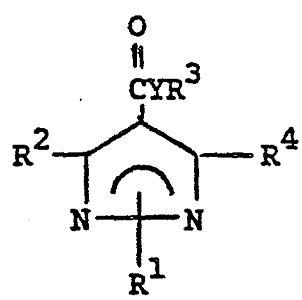

wherein $R^1$ is $C_1$-$C_4$ haloalkyl or $ROR^5$, wherein R is $C_1$-$C_2$ alkylene or $C_2$ alkylidene and $R^5$ is hydrogen, $C_1$-$C_4$ alkyl, $C_2$-$C_5$ alkanoyl, aroyl, $C_8$-$C_{10}$ aralkanoyl, $C_7$-$C_9$ aralkyl, phenyl, or phenyl substituted with $C_1$-$C_4$ alkyl, $C_1$-$C_4$ alkoxy, trihalomethyl, hydroxy, cyano or nitro; or benzoyl substituted with halogen, $C_1$-$C_4$ alkyl, $C_1$-$C_4$ alkoxy, trihalomethyl, hydroxy, cyano or nitro; $R^2$ is $C_1$-$C_4$ alkyl, $C_5$-$C_6$ cycloalkyl, $C_7$-$C_9$ aralkyl, $C_7$-$C_9$ alkaryl, phenyl, naphthyl or a 5- or 6- membered heterocyclic group containing oxygen, sulfur or nitrogen, or $R_2$ is phenyl substituted

with halogen, trihalomethyl, $C_1$-$C_4$ alkoxy, hydroxy or cyano; Y is oxygen or sulfur, $R^3$ is $C_1$-$C_4$ alkyl, $C_2$-$C_4$ alkenyl, $C_3$-$C_4$ alkynyl, $C_2$-$C_6$ alkoxyalkyl or $C_2$-$C_6$ alkylthioalkyl; $R^4$ is hydrogen, $C_1$-$C_4$ alkyl or trihalomethyl; provided that $R^2$ is not 3,4-dialkylphenyl or 3,4 dihalophenyl, are useful as herbicides.

## DETAILED DESCRIPTION

This invention relates to certain pyrazoles useful as herbicides. In particular these inventions relate to pyrazoles having the general formula:

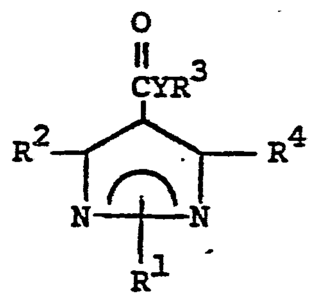

wherein $R^1$ is $C_1$-$C_4$ haloalkyl or $ROR^5$, wherein R is $C_1$-$C_2$ alkylene or $C_2$ alkylidene and $R^5$ is hydrogen, $C_1$-$C_4$ alkyl, $C_2$-$C_5$ alkanoyl, aroyl, $C_8$-$C_{10}$ aralkanoyl, $C_7$-$C_9$ aralkyl, phenyl, or phenyl substituted with $C_1$-$C_4$ alkyl, $C_1$-$C_4$ alkoxy, trihalomethyl, hydroxy, cyano or nitro; or benzoyl substituted with halogen, $C_1$-$C_4$ alkyl, $C_1$-$C_4$ alkoxy, trihalomethyl, hydroxy, gano or nitro; $R^2$ is $C_1$-$C_4$ alkyl, $C_5$-$C_6$ cycloalkyl, $C_7$-$C_9$ aralkyl, $C_7$-$C_9$ alkaryl, phenyl, naphthyl or a 5- or 6- membered heterocyclic group containing oxygen, sulfur or nitrogen, or $R_2$ is phenyl substituted with halogen, trihalomethyl, $C_1$-$C_4$ alkoxy, hydroxy or cyano; Y is oxygen or sulfur, $R^3$ is $C_1$-$C_4$ alkyl, $C_2$-$C_4$ alkenyl, $C_3$-$C_4$ alkynyl, $C_2$-$C_6$ alkoxyalkyl or $C_2$-$C_6$ alkylthioalkyl; $R^4$ is hydrogen, $C_1$-$C_4$ alkyl or trihalomethyl; provided that $R^2$ is not 3,4-dialkylphenyl or 3,4 dihalophenyl. R is preferably methylene.

The pyrazole compounds of the invention may be used for the control of various grasses and broadleaved weeds (including pigweed (*Amaranthus retroflexus* L.), jimsonweed (*Datura stramonium* L.) barnyardgrass (*Echinochloa crusgalli* (L.) Beauv.), and green foxtail (*Setaria viridis* (L.) Beauv.) Application may be in aqueous

solutions or suspensions which may be sprayed onto the weed foliage or the soil surface prior to weed and crop emergence and before or after the crop seed is sown. The compounds of this invention may also be applied by broadcasting of a granular formulation prior to weed and crop emergence.

Compounds of this invention may be added as a "tank mix" to other herbicide solutions so that the number of different weed species controlled in a single application will be increased. The formulations of the compounds of this invention can also include other herbicides so that the spectrum of weeds controlled by spray or granular application may be broadened.

The procedures for using the present substituted pyrazoles of this invention as herbicides can be in accordance with conventional agricultural practice. The chemicals are ordinarily applied as formulations containing a carrier and/or surface-active agent. The formulation can contain one or more of an effective amount of the described substituted pyrazoles if desired; other active herbicides may be included in the formulation as well.

Thus, the pyrazole compounds can be impregnated on finely divided or granular inorganic or organic carriers such as attapulgite clay, sand, vermiculite, corn cobs, activated carbon or other granular carriers known to the art. The impregnated granules can then be spread on the soil. Furthermore, the pyrazole compounds can be formulated as a wettable powder by grinding it into a fine powder and mixing with an inactive powdered carrier to which a surface active and/or dispersing agent has been added. Typical powdered solid carriers are the various mineral silicates, e.g., mica, talc, pyrophyllite and clays. The wettable powder may then be dispersed in water and sprayed on the soil surface or weeds. Similarly, an emulsifiable concentrate may be prepared by dissolving the chemical in a solvent such as benzene, toluene, or other aliphatic or aromatic hydrocarbons to which a surface active dispersing agent has been added. The emulsifiable concentrate may then be dispersed in water and applied by spraying. Suitable surface active agents are well known to those skilled in the art and reference may be had to McCutcheon's Detergents and Emulsifiers, 1980, Allured Publishing Corp., Ridgewood, New Jersey; or Hoffman et

al. U.S. patents 2,614,916, cols. 2 to 4 and 2,547,724 cols. 3 and 4, for examples of appropriate surface active agents which patents are incorporated herein by reference. The concentration of active compound in the formulation may vary widely, e.g., from 1 to 95%. For use as a preemergence herbicide, the compound is applied in a carrier to soil which contains weed and crop seed (either to the surface of the soil or incorporated into the upper 2.5 to 7.6 cm of soil).

The compounds of the present invention are especially active as postemergence herbicides. When used as a postemergence herbicide, the compound is applied in a carrier to the foliage of plants to be controlled. These compounds are most active when applied to dicotyledenous weeds less than 5 cm tall and monocotyledenous weeds less than 12 cm tall.

The most suitable rate of application in any given case will depend on such factors as soil type, soil pH, soil organic matter content, the quantity and intensity of rainfall before and after treatment, the air and soil temperature, light intensity, light duration per day, plant size at time of treatment, and plant species. All of these factors have an influence on the efficiency of the chemicals for use as herbicides. The rate of application can range from 0.5 to 12 kg per hectare; preferably treatment is at about 1 to 6 kg/ha.

The advantages of the novel herbicides of this invention may be more readily appreciated by reference to the following examples:

## EXAMPLE 1

Preparation of Methyl 1-(hydroxmethyl)-3(or 5)-methyl-5(or 3)-phenyl-1H-pyrazole-4-carboxylate.

A mixture of 500 g (2.31 m) of methyl 3(or 5)-methyl-5(or 3)-phenyl-1H-pyrazole-4-carboxylate and 67.0 (2.3 m) of paraformaldehyde in 2.0 liters of dichloromethane was stirred at room temperature for 64 hours and refluxed for 8 hours. An additional 70.0 g (2.33 m) of paraformaldehyde was added and the mixture was stirred at room temperature 56 hours and refluxed 16 hours.

The mixture was filtered, and the solvent was distilled from the filtrate at reduced pressure leaving an oil that was crystallized from ethyl acetate-hexane mixtures. Filtration afforded 445 g of the title compound, m.p. 94-96°C.

| | | |
|---|---|---|
| Analysis (C,H,N); | Calculated: | 63.49; 5.73; 11.38. |
| | Found: | 63.45; 5.67; 11.24. |

EXAMPLE 2

Preparation of Methyl 1-(chloromethyl)-3-(or 5)-methyl-5(or 3)-phenyl-1H-pyrazole-4-carboxylate.

A mixture of 50 g (0.23 mol) of methyl 3(or 5)-methyl-5(or 3)-phenyl-1*H*-pyrazole-4-carboxylate, 28 g (0.93 mol) of paraformaldehyde and about 0.5 g of p-toluenesulfonic acid in 300 ml of dichloromethane was stirred for two days, during which it was heated under reflux for 12 hr. The mixture was filtered and 39 ml (28 g, 0.28 mol) of triethylamine added to the filtrate. The resulting solution was cooled to less than 10°C and treated dropwise with stirring with a solution of 23 ml (37 g, 0.31 mol) of thionyl chloride in 55 ml of dichloromethane. The addition was completed in 1.5 hr. and the stirring continued for 1 hr. at less than 10°C and one day at room temperature. The reaction mixture was then washed with water, dried over anhydrous sodium sulfate and the solvent evaporated. The residue was warmed with toluene, chilled and the title compound, 32 g, m.p. 166-168°C, collected by filtration.

| | | |
|---|---|---|
| Analysis (C,H,N); | Calculated: | 58.98; 4.95; 10.57 |
| | Found: | 58.98; 4.90; 10.23 |

EXAMPLE 3

Preparation of Methyl 1-(2-hydroxyethyl)-5-methyl-3-phenyl-1H-pyrazole-4-carboxylate.

Powdered sodium hydroxide, 28 g (0.70 m) was added portionwise to a well stirred mixture of 80 g (0.68 m) of methyl acetoacetate and 300 ml $CH_2Cl_2$ while the mixture was cooled to less than 10°C. The mixture was stirred in the cold for 6 hours and allowed to stand overnight at room temperature. The mixture was stirred and

cooled to less than 10°C, and 97 g (0.68 m) of benzoyl chloride was added at a rate such that the temperature of the mixture did not exceed 10°C. After the addition was complete the mixture was stirred one hour in the cold and overnight at room temperature. The liquid phase, which was decanted from the salt which had formed, was stirred and cooled to less than 10°C and 200 ml acetic acid was added. To the cold solution was added 51.8 g (0.68 m) of (2-hydroxyethyl)hydrazine, dropwise. The mixture was stirred overnight at room temperature. The mixture was washed twice with water, twice with dilute sodium bicarbonate and twice again with water. The organic phase was dried over anhydrous sodium sulfate, and the solvent was distilled off at reduced pressure leaving an orange oil that crystallized on standing. Recrystallization from an ethyl acetate-hexanes mixture gave 17.1 g of the title product, m.p. 79-80°C.

| | | |
|---|---|---|
| Analysis (C,H,N); | Calculated: | 64.60; 6.20; 10.76. |
| | Found: | 64.94; 6.25; 10.83. |

EXAMPLE 4

Preparation of Methyl 1-(2-chloroethyl)-5-methyl-3-phenyl-1H-pyrazole-4-carboxylate.

To cold (ca.10°C) solution of 5.5 g (0.021 m) of product of Example 3 and 2.5 g (.025 m) of triethylamine in 50 ml $CH_2Cl_2$ was added 3.0 g (.025 m) of thionyl chloride, dropwise. After the addition, the mixture was stirred overnight at ambient temperature. The resulting brown solution was filtered, and the filtrate was washed with water. The organic phase was dried over anhydrous sodium sulfate, and the solvent was distilled off at reduced pressure leaving an oil which was determined to be a mixture of Example 3 product and the above title compound by means of NMR spectroscopy. The oil was taken up again in 50 ml $CH_2Cl_2$ and treated as above with 2.5 g triethylamine of 3.0 g of thionyl chloride. After aqueous work-up, 3.2 g of title product was obtained as a dark brown solid, m.p. 91-93°C.

| | | |
|---|---|---|
| Analysis (C,H,N); | Calculated: | 60.32; 5.42; 10.04 |
| | Found: | 60.35; 5.33; 9.94 |

EXAMPLE 5

Preparation of Methyl 1-[2-(acetyloxy)ethyl]-5-methyl-3-phenyl-1H pyrazole-4-carboxylate.

To a cold (ca.10°C) solution of 5.5 g (0.021 m) of the pyrazole of Example 3 and 2.5 g (0.025 m) of triethylamine in 100 ml of toluene was added 2.0 g (0.025 m) of acetyl chloride, dropwise. After the addition, the mixture was stirred 4 hours at ambient temperature and allowed to stand overnight. The triethylamine hydrochloride, which precipitated, was removed by filtration, and the toluene was distilled off at reduced pressure leaving a colorless oil which solidified on standing. Recrystallization of the product from an ethyl acetate-hexanes mixture afforded 4.9 g of the title compound, m.p. 78-80°C.

| | | |
|---|---|---|
| Analysis (C,H,N); | Calculated: | 63.56; 6.00; 9.27. |
| | Found: | 63.79; 6.24; 9.02. |
| | | 63.60; 5.95; 8.99. |

EXAMPLE 6

Preparation of Methyl 1-(methoxymethyl)-3(or 5)-methyl-5(or 3)-phenyl-1H-pyrazole-4-carboxylate

Methyl 1-(chloromethyl)-3(or 5)-methyl-5(or 3)-phenyl-1H-pyrazole-4-carboxylate (10.0 g, 0.036 mol) was added to a solution of sodium methoxide in methanol (1.5 g of sodium in 60 ml of methanol, prepared under a nitrogen atmosphere) and the mixture heated to 60° with stirring for 3 hr. After the mixture had cooled, it was taken up in dichloromethane (200 ml) and washed with water. The organic phase was dried over anhydrous sodium sulfate and evaporated under reduced pressure to give 8.2 g of the title compound, m.p. 92-94°C.

| | | |
|---|---|---|
| Analysis (C,H,N); | Calculated: | 64.40; 6.20; 10.76. |
| | Found: | 64.62; 6.24; 11.08. |

## EXAMPLE 7

Preparation of Methyl 1-[(acetyloxy)methyl]-3(or 5)-methyl-5(or 3)-phenyl-1H-pyrazole-4-carboxylate

A stirred mixture of 5.0 g (0.020 mol) of methyl 1-(hydroxymethyl)-3(or 5)-methyl-5(or 3)-phenyl-1 *H*-pyrazole-4-carboxylate and 2.4 g (0.024 mol) of triethylamine in toluene (ca 100 ml) was treated dropwise at less than 10°C with 1.9 g (0.024 mol) of acetyl chloride. The mixture was left stirring overnight at room temperature, then the solids were filtered off and the solvent evaporated under reduced pressure. The residue crystallized on standing. Recrystallization from ethyl acetate/hexane gave 4.0 g of the title compound, m.p. 91-94°C.

| | |
|---|---|
| Analysis (C,H,N); | Calculated: 62.49; 5.59; 9.72 |
| | Found:61.56; 6.02; 9.42 |
| | 61.77; 6.16; 9.00 |

According to the general teaching of the above examples the following additional compounds were prepared; they are identified in Table 1.

TABLE I

| Example No. | Compound | Melting Point °C |
|---|---|---|
| 8. | Methyl 3(or 5) (2-chloro-phenyl)-1-(hydroxymethyl)-5(or 3)-methyl-1*H*-Pyrazole-4-carboxylate . | 98-101 |
| 9. | Methyl 1-(hydroxymethyl)-3(or 5)-methyl-5(or 3) (3-methylphenyl)-1*H*-Pyrazole-4-carboxylate | 72-75 |
| 10. | Methyl 3(or 5)-(3-fluorophenyl)-1-(hydroxymethyl)-5(or 3)-methyl-1*H*-Pyrazole-4-carboxylate | 82-84 |
| 11. | Methyl 1-(hydroxmethyl)-3(or 5)-methyl-5(or 3)-[3-(trifluoromethyl)phenyl]-1*H*-Pyrazole-4-carboxylate | 99-101 |
| 12. | Methyl 3(or 5)-(2-fluorophenyl)-1-(hydroxymethyl-5(or 3)-methyl-1*H*- Pyrazole-4-carboxylate | 91-93 |
| 13. | 1,1-Dimethylethyl-1-(hydroxymethyl)-3(or 5)-methyl-5(or 3)-phenyl-1*H*-Pyrazole-4-carboxylate | Oil |

TABLE I (continued)

| Example No. | Compound | Melting Point °C |
|---|---|---|
| 14. | Methyl 1-(hydroxymethyl)-3(or 5)-2-furanyl-5(or 3)-methyl-1H-Pyrazole-4-carboxylate | 104-111 |
| 15. | Ethyl 1-(hydroxymethyl)-3(or 5)-phenyl-5(or 3)-propyl-1H-Pyrazole-4-carboxylate | 61-63 |
| 16. | Methyl 1-(hydroxymethyl)-3(or 5)-methyl-5(or 3)-(2-methylphenyl)-1H-Pyrazole-4-carboxylate | Oil |
| 17. | Methyl 1-(hydroxymethyl)-3(or 5)-phenyl-1H-pyrazole-4-carboxylate | 97-107 |
| 18. | 2-Propenyl 1-(hydroxymethyl)-3(or 5)-methyl-5(or 3)-phenyl-1H-Pyrazole-4-carboxylate | 88-90 |
| 19. | 2-Propynyl 1-(hydroxymethyl)-3(or 5)-methyl-5(or 3)-phenyl-1H-Pyrazole-4-carboxylate | 86-88 |
| 20. | Methyl 1-(benzoyloxy)methyl-3(or 5)-methyl-5(or 3)-phenyl-1H-pyrazole-4-carboxylate | 69-71 |
| 21. | Methyl 3(or 5)-cyclohexyl-1-(hydroxymethyl)-5(or 3)-methyl-1H-Pyrazole-4-carboxylate | 110-112 |
| 22. | Methyl 1-(hydroxymethyl)-3(or 5)-methyl-5(or 3)-(4-methylphenyl)-1H-pyrazole-4-carboxylate | 94-97 |
| 23. | Methyl 3(or 5)-ethyl-1-(hydroxymethyl)-5(or 3)-phenyl-1H-pyrazole-4-carboxylate | 97-98 |
| 24. | Methyl 1-(hydroxymethyl)-3(or 5)-(2-methoxyphenyl)-5(or 3)-methyl-1H-pyrazole-4-carboxylate | 123-125 |
| 25. | 1-Methylpropyl 1-(hydroxymethyl)-3(or 5)-methyl-5(or 3)-methyl-1H-pyrazole-4-carboxylate | Oil |
| 26. | 2-Ethoxyethyl 1-(hydroxymethyl)-3(or 5)-methyl-5(or 3)-phenyl-1H-pyrazole-4-carboxylate | Oil |
| 27. | Methyl 1-(hydroxymethyl)-3(or 5)-phenyl-5(or 3)-propyl-1H-pyrazole-4-carboxylate | 93-94 |
| 28. | Methyl 3(or 5)-(2-bromophenyl)-1-(hydroxymethyl)-5(or 3)-methyl-1H-pyrazole-4-carboxylate | 90-98 |
| 29. | Methyl 3(or 5)-(4-bromophenyl)-1-(hydroxymethyl)-5(or 3)-methyl-1H-pyrazole-4-carboxylate | 111-120 |
| 30. | Methyl 3(or 5)-(3-bromophenyl)-1-(hydroxymethyl)-5(or 3)-methyl-1H-pyrazole-4-carboxylate | 96-102 |

TABLE I (continued)

| Example No. | Compound | Melting Point °C |
|---|---|---|
| 31. | Methyl 1-(hydroxymethyl)-3(or 5)-methyl-5(or 3)-(1-naphthalenyl)-1H-pyrazole-4-carboxylate | 97-107 |
| 32. | Methyl 3(or 5)-methyl-1-(phenoxymethyl)-5(or 3)-phenyl-1H-pyrazole-4-carboxylate | 58-61 |
| 33. | Ethyl 1-(ethoxymethyl)-3(or 5)-methyl-5(or 3)-(3-methylphenyl)-1H-pyrazole-4-carboxylate | Oil |
| 34. | Methyl 3(or 5)(4-chlorophenyl-(1-hydroxymethyl)-5 (or 3)-methyl-1H-Pyrazole-4-carboxylate | 113-116 |
| 35. | Methyl 1-(chloromethyl)-3(or 5)-methyl-5(or 3)-(3-methylphenyl)-1H-Pyrazole-4-carboxylate | 127-129 |

EXAMPLE 36

To illustrate effectiveness of the described pyrazole derivatives as preemergence herbicides, 600 mg chemical was dissolved in 10 ml organic solvent (e.g., acetone) to which 30 mg conventional emulsifying agent (e.g., ethoxylated sorbitan monolaurate "Tween 20") was added. The solution was diluted to 100 ml with distilled water. Twenty milliliters of this 6000 ppm solution was diluted to 250 ppm with distilled water. The chemical was applied at the rate of 11.2 kg/ha (kilograms per hectare) by drenching 46 ml of the 250 ppm solution on the surface of soil in 11.4 cm diameter plastic pots which had been sown with the following weed seeds: velvetleaf (Abutilon theophiasti Medic.) jimsonweed (Datura stramonium L.), tall morningglory (Ipomea purpurea (L.) Roth), switchgrass (Panicum virgatum L.), barnyardgrass (Echinochloa crusgalli (L.) Beauv.) and green foxtail (Setaria viridis (L.) Beauv). The percent control of the weeds compared to untreated checks was determined two weeks after treatment. Table II shows the results with the preemergence herbicides of the invention prepared in accordance with the above samples.

EXAMPLE 37

To illustrate effectiveness of the described pyrazole derivatives as postemergence herbicides, the 6000 ppm solution described under

Example 36 was atomized with a number 152 DeVilbiss sprayer, wetting the foliage to the drip point. The weeds, which are the same species as described under Example 36, were treated six days after emergence. The percent control was evaluated two weeks after treatment. Table III shows the results with the postemergence herbicides of the invention.

EXAMPLE 38

To illustrate efficacy at 11.2 kg/ha preemergence, 250 ppm solutions were drenched onto the soil surface at the rate of 80 ml per 15.2 cm diameter pot. A rate of 1.12 kg/ha required 25 ppm. The seeds of several weed species were sown into the soil of each pot prior to chemical application. The percent control of weeds compared to untreated checks was determined two weeks after emergence. Table IV shows the results of efficacy tests at rates of 11.2 and 1.12 kg/ha.

EXAMPLE 39

To illustrate efficacy postemergence at rates less than 6000 ppm, the solutions described in Example 36 were diluted to 4000, 2000, 1000, 500, 250 and 125 ppm with distilled water and applied to the plant foliage as described in Example 2. TABLE V illustrates the results of postemergence application at rates lower than 6000 ppm.

EXAMPLE 40

Selectivity of a herbicide is desirable since it allows control of weeds growing among desirable crop plants. To illustrate the usefulness of the compounds of this invention as selective postemergence herbicides, 400 mg chemical was dissolved in 10 ml organic solvent such as acetone containing 25 mg conventional emulsifying agent (e.g., ethoxylated sorbitan monolaurate "Tween 20" [trademark]) and diluted to 50 ml with distilled water. This solution was sprayed at the rate of 2.24 kg active in 280 liters of water per hectare onto the foliage of plants growing in 15" x 20" flats. Lower rates were achieved by dilution with distilled water. For example, the solution for 2.24 kg/ha diluted 50% with water produced the solution for 1.12 kg/ha. The percent weed control and crop injury were evaluated three weeks after treatment. TABLE VI illustrates the usefulness of these chemicals as selective postemergence herbicides.

TABLE II

PERCENT WEED CONTROL AT 11.2 kg/ha, PREEMERGENCE

Percent Weed Control

| Example No. | Velvet Leaf | Jimson Weed | Wild Morning Glory | Barnyard Grass | Switch Grass | Green Foxtail |
|---|---|---|---|---|---|---|
| 1 | 100 | 50 | 80 | 100 | 90 | 100 |
| 2 | 100 | 75 | 100 | 100 | 100 | 100 |
| 4 | 0 | 0 | 0 | 0 | 0 | 50 |
| 6 | 100 | 0 | 100 | 80 | 35 | 100 |
| 7 | 100 | 75 | 100 | 100 | 95 | 100 |
| 8 | 90 | 0 | 60 | 0 | 0 | 0 |
| 9 | 100 | 0 | 0 | 90 | 10 | 40 |
| 10 | 100 | 45 | 100 | 100 | 85 | 90 |
| 11 | 100 | 0 | 80 | 20 | 25 | 95 |
| 12 | 95 | 0 | 0 | 90 | 80 | 95 |
| 16 | 100 | 25 | 50 | 80 | 90 | 75 |
| 19 | 50 | 100 | 0 | 100 | 100 | 100 |
| 20 | 100 | 50 | 100 | 100 | 95 | 100 |
| 21 | 100 | 95 | 100 | 100 | 100 | 80 |
| 23 | 90 | 0 | 100 | 100 | 100 | 100 |
| 33 | 0 | 0 | 0 | 95 | 0 | 65 |
| 35 | 50 | 0 | 65 | 100 | 100 | 100 |

TABLE III

PERCENT WEED CONTROL AT 6000 PPM, POSTEMERGENCE

Percent Weed Control

| Example No. | Velvet Leaf | Jimson Weed | Wild Morning Glory | Barnyard Grass | Switch Grass | Green Foxtail |
|---|---|---|---|---|---|---|
| 1 | 100 | 5 | 100 | 100 | 15 | 75 |
| 2 | 30 | 80 | 100 | 60 | 90 | 85 |
| 3 | 5 | 5 | 25 | 40 | 0 | 20 |
| 4 | 0 | 0 | 45 | 0 | 0 | 5 |
| 5 | 0 | 0 | 5 | 5 | 0 | 5 |
| 6 | 95 | 0 | 100 | 30 | 0 | 10 |
| 7 | 75 | 25 | 100 | 90 | 100 | 100 |
| 8 | 70 | 25 | 70 | 30 | 0 | 30 |
| 9 | 100 | 35 | 100 | 100 | 100 | 95 |
| 10 | 100 | 90 | 100 | 100 | 90 | 100 |
| 11 | 100 | 50 | 100 | 100 | 35 | 95 |
| 12 | 100 | 40 | 100 | 100 | 70 | 85 |
| 13 | 0 | 0 | 5 | 25 | 0 | 15 |
| 14 | 100 | 5 | 95 | 90 | 15 | 90 |
| 15 | 5 | 5 | 30 | 45 | 0 | 15 |
| 16 | 95 | 90 | 100 | 90 | 100 | 100 |
| 18 | 10 | 95 | 100 | 100 | 80 | 100 |
| 19 | 100 | 100 | 100 | 90 | 100 | 100 |
| 20 | 20 | 10 | 100 | 90 | 100 | 100 |
| 21 | 100 | 100 | 100 | 100 | 100 | 100 |

TABLE III (Continued)

PERCENT WEED CONTROL AT 6000 PPM, POSTEMERGENCE

Percent Weed Control

| Example No. | Velvet Leaf | Jimson Weed | Wild Morning Glory | Barnyard Grass | Switch Grass | Green Foxtail |
|---|---|---|---|---|---|---|
| 22 | 45 | 5 | 100 | 90 | 0 | 75 |
| 23 | 100 | 75 | 100 | 95 | 50 | 80 |
| 24 | 5 | 0 | 40 | 15 | 0 | 5 |
| 25 | 0 | 0 | 0 | 15 | 0 | 5 |
| 26 | 5 | 0 | 20 | 40 | 100 | 100 |
| 27 | 5 | 5 | 35 | 45 | 100 | 100 |
| 33 | 5 | 0 | 95 | 60 | 100 | 100 |
| 34 | 25 | 50 | 100 | 80 | 0 | 90 |
| 35 | 100 | 80 | 100 | 85 | 90 | 70 |

TABLE IV

PREEMERGENCE WEED CONTROL

Percent Weed Control or Crop Injury

| Example No. | Rate Kg/Ha | Jimson Weed | Pig Weed | Morning Glory | Wild Oats | Green Foxtail | Barnyard Grass |
|---|---|---|---|---|---|---|---|
| 1 | 11.2 | 100 | 100 | 100 | 95 | 100 | 100 |
| | 1.12 | 40 | 100 | 20 | 30 | 95 | 50 |

TABLE V

POSTEMERGENCE WEED CONTROL

Percent Weed Control or Crop Injury

| Example No. | Rate PPM | Jimson Weed | Pig Weed | Morning Glory | Wild Oats | Green Foxtail |
|---|---|---|---|---|---|---|
| 1 | 1000 | 75 | 100 | 90 | 90 | 100 |
| | 500 | 0 | 20 | 50 | 50 | 100 |
| | 250 | 0 | 20 | 50 | 50 | 100 |
| 22 | 4000 | 60 | 60 | 90 | 10 | 50 |
| | 2000 | 0 | 50 | 20 | 0 | 20 |
| | 1000 | 0 | 0 | 10 | 0 | 10 |
| 34 | 4000 | 30 | 100 | 100 | 30 | 100 |
| | 2000 | 25 | 100 | 35 | 25 | 100 |
| | 1000 | 10 | 100 | 20 | 20 | 100 |

| Example No. | Rate PPM | Barnyard Grass | Corn | Soy-bean | Rice | Cotton |
|---|---|---|---|---|---|---|
| 1 | 1000 | 100 | 100 | 95 | 60 | 10 |
| | 500 | 100 | 10 | 10 | 10 | 5 |
| | 250 | 80 | 10 | 10 | 10 | 10 |
| 22 | 4000 | 90 | 30 | 15 | 20 | 0 |
| | 2000 | 20 | - | 10 | 20 | 0 |
| | 1000 | 20 | - | 10 | 10 | 0 |
| 34 | 4000 | 85 | 30 | 10 | 20 | 0 |
| | 2000 | 75 | 30 | 10 | 20 | 0 |
| | 1000 | 50 | - | 10 | 20 | 0 |

TABLE VI: POSTEMERGENCE SELECTIVITY TEST

Percent Weed Control or Crop Injury

| Example No. | Rate Kg/Ha | Wheat | Barley | Oats | Sun-Flower |
|---|---|---|---|---|---|
| 1 | 2.24 | 35 | 20 | 70 | 0 |
| | 1.12 | 25 | 0 | 30 | 0 |
| | 0.28 | 0 | 0 | 0 | 0 |

| Example No. | Rate Kg/ha | Sugar beet | Corn | Cotton | Soy-bean |
|---|---|---|---|---|---|
| 1 | 2.24 | 100 | 0 | 100 | 60 |
| | 1.12 | 100 | 0 | 100 | 15 |
| | 0.28 | 100 | 0 | 70 | 0 |

| Example No. | Rate Kg/Ha | Sorghum | Cow Pea | Pinto Beans |
|---|---|---|---|---|
| 1 | 2.24 | 0 | 70 | 80 |
| | 1.12 | 0 | 60 | 0 |
| | 0.28 | 0 | 0 | 0 |

| Example No. | Rate Kg/Ha | Rice | Cucumber | Alfalfa | Tomato |
|---|---|---|---|---|---|
| 1 | 2.24 | 70 | 100 | 100 | 100 |
| | 1.12 | 70 | 100 | 100 | 50 |
| | 0.28 | 0 | 100 | 100 | 50 |

| Example No. | Rate Kg/Ha | Velvet Leaf | Sickle Pod | Morning Glory |
|---|---|---|---|---|
| 1 | 2.24 | 95 | 75 | 85 |
| | 1.12 | 95 | 75 | 85 |
| | 0.28 | 90 | 50 | 70 |

It is evident from the preceding examples that the preferred pyrazole compounds for use as preemergent herbicides are the compounds of Examples 1, 10 and 11. The preferred pyrozoles for use as post emergence herbicides are the compounds of Examples 1, 9, 10, 11, 12, 14 and 18.

Additional pyrazole-4-carboxylates which have herbicidal activity and are within the scope of the invention are: Methyl 1-(butoxymethyl)-3(or 5)-methyl-5(or 3)-phenyl-1 H-pyrazole-4-carboxylate; S-methyl 1-[(1-oxybutoxy)methyl]-3(or 5)-methyl-5(or 3)-phenyl-1H-pyrazole-4-carbothioate; 2-(methylthio)ethyl 1-(1-oxo-2-phenylethoxy)-3(or 5)-methyl-5(or 3)-phenyl-1 H-pyrazole-4-carboxylate; Methyl 1-(hydroxymethyl)-3(or 5)-phenyl-5(or 3)-trifluoromethyl)-1 H-pyrazole-4-carboxylate; Methyl 1-[[[4-(1,1-dimethylethyl)benzoyl]oxy]methyl]-3(or 5)-methyl-5(or 3)-(phenylmethyl)-1H-pyrazole-4-carboxylate; Methyl 3(or 5)-(3-butylphenyl)-5(or 3)-methyl-1-[[(4-propoxybenzoyl)oxy]methyl]-1 H-pyrazole-4-carboxylate; Methyl 3(or 5)-methyl-5(or 3)-(3-thienyl)-1-[[3-(trifluoromethyl)benzoyl]oxy]methyl]-1 H-pyrazole-4-carboxylate; Methyl 1-[[(2-hydroxybenzoyl)oxy]methyl]-3(or 5)-methyl-5(or 3)-(4-morpholinyl)-1 H-pyrazole-4-carboxylate; Methyl 1-[[(3-cyanobenzoyl)-oxy]methyl]-3(or 5)-(3-cyanophenyl)-5(or 3)-methyl-1H-pyrazole-4-carboxylate; Methyl 3(or 5)-(3-butoxyphenyl)-5(or 3)-methyl-1-[[(4-nitrobenzoyl)oxy]methyl]-1 H-pyrazole-4-carboxylate; Methyl 1-(hydroxymethyl)-3(or 5)-hydroxyphenyl)-5(or 3)-methyl-1H-pyrazole-4-carboxylate; Methyl 1-[(4-hydroxyphenoxy)methyl]-3(or 5)-methyl-5(or 3)-phenyl-1 H-pyrazole-4-carboxylate; Methyl 1-[(4-cyanophenoxy)methyl]-3(or 5)-methyl-5(or 3)-phenyl-1H-pyrazole-4-carboxylate; Methyl 3(or 5)-methyl-5(or 3)-phenyl-1-[[3-(trifluoromethyl)phenoxy]methyl]-1H-pyrazole-4-carboxylate.

CLAIMS:

1. A pyrazole compound having the structural formula

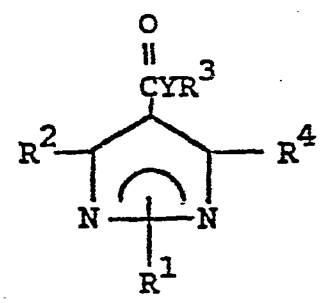

wherein $R^1$ is $C_1$-$C_4$ haloalkyl or $ROR^5$, wherein R is $C_1$-$C_2$ alkylene or $C_2$ alkylidene and $R^5$ is hydrogen, $C_1$-$C_4$ alkyl, $C_2$-$C_5$ alkanoyl, aroyl, $C_8$-$C_{10}$ aralkanoyl, $C_7$-$C_9$ aralkyl, phenyl, or phenyl substituted with $C_1$-$C_4$ alkyl, $C_1$-$C_4$ alkoxy, trihalomethyl, hydroxy, cyano or nitro; or benzoyl substituted with halogen, $C_1$-$C_4$ alkyl, $C_1$-$C_4$ alkoxy, trihalomethyl, hydroxy, gano or nitro; $R^2$ is $C_1$-$C_4$ alkyl, $C_5$-$C_6$ cycloalkyl, $C_7$-$C_9$ aralkyl, $C_7$-$C_9$ alkaryl, phenyl, naphthyl or a 5- or 6- membered heterocyclic group containing oxygen, sulfur or nitrogen, or $R_2$ is phenyl substituted with halogen, trihalomethyl, $C_1$-$C_4$ alkoxy, hydroxy or cyano; Y is oxygen or sulfur, $R^3$ is $C_1$-$C_4$ alkyl, $C_2$-$C_4$ alkenyl, $C_3$-$C_4$ alkynyl, $C_2$-$C_6$ alkoxyalkyl or $C_2$-$C_6$ alkylthioalkyl; $R^4$ is hydrogen, $C_1$-$C_4$ alkyl or trihalomethyl; provided that $R^2$ is not 3,4-dialkylphenyl or 3,4 dihalophenyl.

2. The compound of claim 1 wherein $R^1$ is $C_1$-$C_2$ chloroalkyl; R is $C_1$-$C_2$ alkylene, $R^5$ is hydrogen, $C_1$-$C_4$ alkyl, phenyl or benzyl; $R^2$ is cyclohexyl, phenyl, halophenyl, trifluoromethylphenyl, methoxyphenyl, benzyl, naphthyl, thienyl or furanyl; Y is oxygen or sulfur; $R^3$ is $C_1$-$C_2$ alkyl, propenyl or propynyl; $R^4$ is hydrogen or $C_1$-$C_4$ alkyl.

3. The compound of claim 1 wherein $R^1$ is selected from chloromethyl, chloroethyl, hydroxymethyl, hydroxyethyl, methoxyethyl, ethoxymethyl, phenoxymethyl, methylcarbonyloxymethyl and benzoyloxymethyl; $R^2$ is cyclohexyl, phenyl, naphthyl, furanyl or phenyl substituted with fluorine, chlorine, bromine, methyl, trifluoromethyl or methoxy; $R^3$ is $C_1$-$C_3$ alkyl, allyl, propargyl or ethoxyethyl; $R^4$ is hydrogen or $C_1$-$C_3$ alkyl; and Y is oxygen.

4. A preemergence herbicidal composition comprising a pyrazole compound having the structural formula

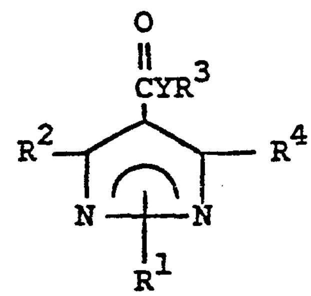

wherein $R^1$ is chloromethyl, chloroethyl, hydroxymethyl, methoxymethyl, ethoxymethyl, phenoxymethyl, or benzoyloxymethyl; $R^2$ is cyclohexyl, phenyl, naphthyl, or phenyl substituted with fluorine, chlorine, bromine or methyl, provided that such substituents are not in the para-position and $R^2$ is not 3,4-dialkylphenyl or 3,4-dihalophenyl; $R^3$ is $C_1$-$C_2$ alkyl, or propargyl; $R^4$ is hydrogen or $C_1$-$C_2$ alkyl and Y is oxygen, and a carrier therefor.

5. The composition according to claim 4 wherein the pyrazole compound is selected from the group consisting of methyl 1-(hydroxymethyl)-3 (or 5) methyl-5(or 3)-phenyl-1H-pyrazole-4-carboxylate; methyl-3(or 5)-(3-fluorophenyl)-1-(hydroxymethyl)-5(or 3)-methyl-, 1 H-pyrazole-4-carboxylate; methyl-1-(Hydroxymethyl)-3(or 5)-methyl-5(or 3)-[3-(trifluoromethyl)phenyl]-1 H-pyrazole-4-carboxylate or mixtures thereof.

6. A postemergence herbicidal composition comprising a pyrazole compound having the structural formula

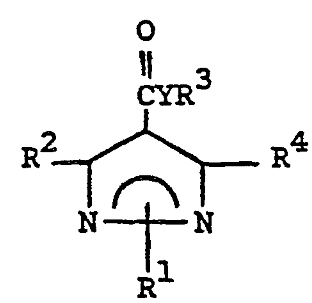

wherein $R^1$ is $C_1$-$C_4$ haloalkyl or $ROR^5$, wherein R is $C_1$-$C_2$ alkylene or $C_2$ alkylidene and $R^5$ is hydrogen, $C_1$-$C_4$ alkyl, $C_2$-$C_5$ alkanoyl, aroyl, $C_8$-$C_{10}$ aralkanoyl, $C_7$-$C_9$ aralkyl, phenyl, or

phenyl substituted with $C_1$-$C_4$ alkyl, $C_1$-$C_4$ alkoxy, trihalomethyl, hydroxy, cyano or nitro; or benzoyl substituted with halogen, $C_1$-$C_4$ alkyl, $C_1$-$C_4$ alkoxy, trihalomethyl, hydroxy, gano or nitro; $R^2$ is $C_1$-$C_4$ alkyl, $C_5$-$C_6$ cycloalkyl, $C_7$-$C_9$ aralkyl, $C_7$-$C_9$ alkaryl, phenyl, naphthyl or a 5- or 6- membered heterocyclic group containing oxygen, sulfur or nitrogen, or $R_2$ is phenyl substituted with halogen, trihalomethyl, $C_1$-$C_4$ alkoxy, hydroxy or cyano; Y is oxygen or sulfur, $R^3$ is $C_1$-$C_4$ alkyl, $C_2$-$C_4$ alkenyl, $C_3$-$C_4$ alkynyl, $C_2$-$C_6$ alkoxyalkyl or $C_2$-$C_6$ alkylthioalkyl; $R^4$ is hydrogen, $C_1$-$C_4$ alkyl or trihalomethyl; provided that $R^2$ is not 3,4-dialkylphenyl or 3,4 dihalophenyl.

7. The composition according to claim 6 wherein $R^1$ is chloromethyl, R is methylene, $R^5$ is hydrogen or methylcarbonyl, $R^2$ is cyclohexyl, phenyl, 2- or 3-fluorophenyl, 2- or 3-methylphenyl or 2- or 3-trifluoromethylphenyl; $R^3$ is methyl, allyl or propargyl, $R^4$ is methyl or ethyl; and Y is oxygen.

8. A postemergence composition according to claim 6 wherein the pyrazole compound is Methyl 1-(hydroxymethyl)-3(or 5)-methyl-5(or 3) (3-methylphenyl)-1 <u>H</u>-Pyrazole-4-carboxylate; Methyl 3(or 5)-(3-fluorophenyl)-1-(hydroxymethyl)-5(or 3)-methyl-1 <u>H</u>-Pyrazole-4-carboxylate; Methyl 1-(hydroxmethyl)-3(or 5)-methyl-5(or 3)-[3-(trifluoromethyl)phenyl]-1 <u>H</u>-Pyrazole-4-carboxylate; Methyl 3(or 5)-(2-fluorophenyl)-1-(hydroxymethyl-5(or 3)-methyl-1 <u>H</u>- Pyrazole-4-carboxylate; Methyl 1-(hydroxymethyl)-3(or 5)-2-furanyl-5(or 3)-methyl-1<u>H</u>-Pyrazole-4-carboxylate; Methyl 1-(hydroxymethyl)-3(or 5)-methyl-5(or 3)-(2-methylphenyl)-1 <u>H</u>-Pyrazole-4-carboxylate; 2-Propenyl 1-(hydroxymethyl)-3(or 5)-methyl-5(or 3)-phenyl-1 <u>H</u>-Pyrazole-4-carboxylate; 2-Propynyl 1-(hydroxymethyl)-3(or 5)-methyl-5(or 3)-phenyl-1 <u>H</u>-Pyrazole-4-carboxylate; Methyl 3(or 5)-cyclohexyl-1-(hydroxymethyl)-5(or 3)-methyl-1 <u>H</u>-Pyrazole-4-carboxylate; Methyl 3(or 5)-ethyl-1-(hydroxymethyl)-5(or 3)-phenyl-1 <u>H</u>-pyrazole-4-carboxylate; Methyl 1-(chloromethyl)-3(or 5)-methyl-5(or 3)-(3-methylphenyl)-1 <u>H</u>-Pyrazole-4-carboxylate; Methyl 1-(hydroxymethyl)-3(or 5)-methyl-5(or 3)-phenyl-1 <u>H</u>-pyrazole-4-carboxylate; Methyl 1-[(acetyloxy)methyl]-3(or 5)-methyl-5(or 3)-phenyl-1<u>H</u>-pyrazole-4-carboxylate or mixtures thereof.

9. The composition of claims 4 or 7 wherein the pyrazole compound is impregnated on a finely divided, powdered or granular carrier.

10. The composition of claim 9 wherein the carrier is selected from the group consisting of attapulgite clay, sand, vermiculite, corn cobs, activated charcoal or mixtures thereof.

11. The composition of claim 9 wherein surface active or dispersing agents are added and the carrier is a mineral silicate in powdered form.

12. The composition of claim 11 wherein the mineral silicate is mica, talc, pyrophyllite or clay.

13. A method for preemergence control of weed which comprises treating the area to be controlled with an effective amount of a composition comprising a pyrazole compound having the general formula

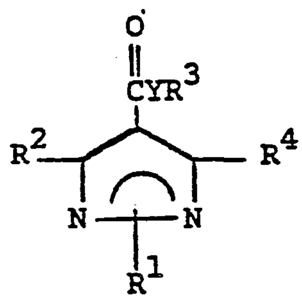

wherein $R^1$ is chloromethyl, chloroethyl, hydroxymethyl, methoxymethyl, ethoxymethyl, phenoxymethyl, or benzoyloxymethyl; $R^2$ is cyclohexyl, phenyl, or phenyl substituted with fluorine, chlorine, bromine or methyl, provided that such substituents are not in the para-position and $R^2$ is not 3,4-dialkylphenyl or 3,4-dichlorophenyl; $R^3$ is $C_1$-$C_2$ alkyl, or propargyl; $R^4$ is hydrogen or $C_1$-$C_2$ alkyl and Y is oxygen, and a carrier therefor.

14. The method according to claim 13 wherein the pyrazole compound is selected from the group consisting of methyl 1-(hydroxy-methyl)-3(or 5) methyl-5(or 3)-phenyl-1 H-pyrazole-4-carboxylate; methyl-3(or 5)-(3-fluorophenyl)-1-(hydroxymethyl)-5(or 3)-methyl-1 H-pyrazole-4-carboxylate; methyl-1-(Hydroxymethyl)-3(or 5)-methyl-5(or 3)-(3-(trifluoromethyl)phenyl)-1 H-pyrazole-4-carboxylate or mixtures thereof.

15. A method for the postemergence control of weeds which comprises treating the area to be controlled with an effective amount of a composition having the structural formula:

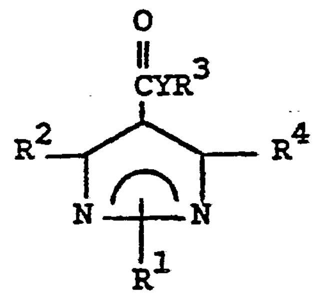

wherein $R^1$ is $C_1$-$C_4$ haloalkyl or $ROR^5$, wherein R is $C_1$-$C_2$ alkylene or $C_2$ alkylidene and $R^5$ is hydrogen, $C_1$-$C_4$ alkyl, $C_2$-$C_5$ alkanoyl, aroyl, $C_8$-$C_{10}$ aralkanoyl, $C_7$-$C_9$ aralkyl, phenyl, or phenyl substituted with $C_1$-$C_4$ alkyl, $C_1$-$C_4$ alkoxy, trihalomethyl, hydroxy, cyano or nitro; or benzoyl substituted with halogen, $C_1$-$C_4$ alkyl, $C_1$-$C_4$ alkoxy, trihalomethyl, hydroxy, gano or nitro; $R^2$ is $C_1$-$C_4$ alkyl, $C_5$-$C_6$ cycloalkyl, $C_7$-$C_9$ aralkyl, $C_7$-$C_9$ alkaryl, phenyl, naphthyl or a 5- or 6- membered heterocyclic group containing oxygen, sulfur or nitrogen, or $R_2$ is phenyl substituted with halogen, trihalomethyl, $C_1$-$C_4$ alkoxy, hydroxy or cyano; Y is oxygen or sulfur, $R^3$ is $C_1$-$C_4$ alkyl, $C_2$-$C_4$ alkenyl, $C_3$-$C_4$ alkynyl, $C_2$-$C_6$ alkoxyalkyl or $C_2$-$C_6$ alkylthioalkyl; $R^4$ is hydrogen, $C_1$-$C_4$ alkyl or trihalomethyl; provided that $R^2$ is not 3,4-dialkylphenyl or 3,4 dihalophenyl.

16. The method according to claim 15 wherein the pyrazole compound is Methyl 1-(hydroxymethyl)-3(or 5)-methyl-5(or 3) (3-methylphenyl)-1 H-Pyrazole-4-carboxylate; Methyl 3(or 5)-(3-fluorophenyl)-1-(hydroxymethyl)-5(or 3)-methyl-1 H-Pyrazole-4-carboxylate; Methyl 1-(hydroxmethyl)-3(or 5)-methyl-5(or 3)-[3-(trifluoromethyl)phenyl]-1 H-Pyrazole-4-carboxylate; Methyl 3(or 5)-(2-fluorophenyl)-1-(hydroxymethyl-5(or 3)-methyl-1 H- Pyrazole-4-carboxylate; Methyl 1-(hydroxymethyl)-3(or 5)-2-furanyl-5(or 3)-methyl-1 H-Pyrazole-4-carboxylate; Methyl 1-(hydroxymethyl)-3(or 5)-methyl-5(or 3)-(2-methylphenyl)-1 H-Pyrazole-4-carboxylate; 2-Propenyl 1-(hydroxymethyl)-3(or 5)-methyl-5(or 3)-phenyl-1 H-Pyrazole-4-carboxylate; 2-Propynyl 1-(hydroxymethyl)-3(or

5)-methyl-5(or 3)-phenyl-1 H-Pyrazole-4-carboxylate; Methyl 3(or 5)-cyclohexyl-1-(hydroxymethyl)-5(or 3)-methyl-1 H-Pyrazole-4-carboxylate; Methyl 3(or 5)-ethyl-1-(hydroxymethyl)-5(or 3)-phenyl-1 H-pyrazole-4-carboxylate; Methyl 1-(chloromethyl)-3(or 5)-methyl-5(or 3)-(3-methylphenyl)-1 H-Pyrazole-4-carboxylate; Methyl 1-(hydroxymethyl)-3(or 5)-methyl-5(or 3)-phenyl-1 H-pyrazole-4-carboxylate; Methyl 1-[(acetyloxy)methyl]-3(or 5)-methyl-5(or 3)-phenyl-1H-pyrazole-4-carboxylate or mixtures thereof.

17. The method of claims 14 or 15 wherein the pyrazole compound is impregnated on a finely divided, powdered or granular carrier or mixture thereof.

18. The method of claim 16 wherein the carrier is selected from the group consisting of attapulgite clay, sand, vermiculite, corn cobs, activated charcoal or mixtures thereof.

19. The method of claim 16 wherein surface active or despersing agents are added and the carrier is of a mineral silicate in powdered form.

20. The method of claim 18 wherein the mineral silicate is mica, talc, pyrophyllite or clay.